(19) Europäisches Patentamt
European Patent Office
Office européen des brevets

(11) Publication number: **0 058 435**
**B1**

(12) **EUROPEAN PATENT SPECIFICATION**

(45) Date of publication of patent specification: **30.12.86**

(21) Application number: **82101233.3**

(22) Date of filing: **18.02.82**

(51) Int. Cl.$^4$: **C 07 D 233/70,**
C 07 D 405/06,
C 07 D 409/06, A 61 K 31/415

(54) **Novel 4-aroylimidazol-2-ones.**

(30) Priority: **18.02.81 US 235453**

(43) Date of publication of application:
**25.08.82 Bulletin 82/34**

(45) Publication of the grant of the patent:
**30.12.86 Bulletin 86/52**

(84) Designated Contracting States:
**AT BE CH DE FR IT LI LU NL SE**

(58) References cited:
**FR-A-2 459 233**

**The file contains technical information submitted after the application was filed and not included in this specification**

(73) Proprietor: **MERRELL DOW PHARMACEUTICALS INC.**
**2110 E. Galbraith Road**
**Cincinnati Ohio 45215 (US)**

(72) Inventor: **Schnettler, Richard A.**
**9874 Forest Glen Drive**
**Cincinnati, OH 45242 (US)**
Inventor: **Dage, Richard C.**
**7825 Shadowhill Way**
**Cincinnati, OH 45242 (US)**
Inventor: **Grisar, Martin J.**
**9, rue Bain Finkwiller**
**F-67000 Strasbourg (FR)**

(74) Representative: **Vossius & Partner**
**Siebertstrasse 4 P.O. Box 86 07 67**
**D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 058 435 B1**

**Description**

This invention relates to 4-aroylimidazol-2-ones; their use as antihypertensives, cardiotonics and antithrombotics; their pharmaceutical compositions; and their preparation.

The closest prior art known to the applicants is found in U.S. patents 2,514,380 and 2,441,933, as well as in R. Duschinsky and L. A. Dolan, *J. Am. Chem. Soc. 68,* 2350—55 (1946); *ibid. 70,* 657—62 (1948); *ibid. 67, 2079—84* (1945); and Y. A. Rozin, E. P. Dorienko and Z. V. Pushkareva, *Khim, Geterotsikl. Soedin., 4*(4), 698—701 (1968). These references disclose the preparation and chemical intermediate utility of the following compounds;

4-benzoyl-1,3-dihydro-2*H*-imidazol-2-one;
4-benzoyl-1,3-diacetyl-1,3-dihydro-2*H*-imidazol-2-one;
4-benzoyl-1,3-dihydro-5-(lower alkyl)-2*H*-imidazol-2-one;
4-benzozyl-1,3-diacetyl-1,3-dihydro-5-methyl-2*H*-imidazol-2-one;
1,3-diacetyl-1,3-dihydro-4-(3,4-dimethylbenzoyl)-2*H*-imidazol-2-one;
1,3-dihydro-4-(hydroxybenzoyl)-2*H*-imidazol-2-one;
1,3-dihydro-4-(hydroxybenzoyl)-5-(lower alkyl)-2*H*-imidazol-2-one;
1,3-dihydro-4-(3,4-dihydroxybenzoyl)-2*H*-imidazol-2-one;
1,3-dihydro-4-(4-nitrobenzoyl)-2*H*-imidazol-2-one;
1,3-dihydro-4-methyl-5-(4-nitrobenzoyl)-2*H*-imidazol-2-one,
4-(3-aminobenzoyl)-1,3-dihydro-2*H*-imidazol-2-one,
4-(4-aminobenzoyl)-1,3-dihydro-2*H*-imidazol-2-one, and
4-(4-aminobenzoyl)-1,3-dihydro-5-methyl-2*H*-imidazol-2-one.

4-roylimidazol-2-ones and their pharmaceutical use are known from FR—A—2,459,233.

This invention is directed to pharmaceutically active 4-aroylimidazol-2-ones of general Formula 1

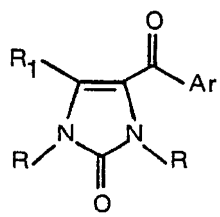

Formula 1

wherein Ar is phenyl monosubstituted at the ortho, meta or para position with $X_1$; $X_1$ is a straight or branched chain lower alkylsulfoxide of from 1 to 4 carbon atoms or a straight or branched chain lower alkylsulfone of from 1 to 4 carbon atoms; R is hydrogen, a straight or branched chain lower alkyl of from 1 to 4 carbon atoms, a straight or branched chain lower alkylcarbonyl of from 1 to 4 carbon atoms, or a benzoyl group; $R_1$ is hydrogen or a straight or branched chain lower alkyl of from 1 to 4 carbon atoms; or a pharmaceutically acceptable salt thereof. These compounds are useful as antihypertensives, cardiotonics and antithrombotics. This invention is directed, furthermore, to the process of preparing the 4-aroylimidazol-2-ones as well as their pharmaceutical compositions.

Illustrative examples of a straight or branched chain lower alkyl of from 1 to 4 carbon atoms as used herein are methyl, ethyl, *n*-propyl, isopropyl, *n*-butyl and isobutyl.

As used herein, the term halide is taken to mean fluoride, chloride, bromide, or iodide.

As used herein the term "a straight or branched chain lower alkylsulfoxide of from 1 to 4 carbon atoms" is taken to mean a group of the structure, S(O)alkyl, wherein the alkyl moiety is a straight or branched chain alkyl of from 1 to 4 carbon atoms and may be, for example, methyl, ethyl, *n*-propyl, isopropyl, *n*-butyl or isobutyl.

As used herein, the term "a straight or branched chain lower alkylsulfone of from 1 to 4 carbon atoms" is taken to mean a group of the structure, $S(O)_2$alkyl, wherein the alkyl moiety is a straight or branched chain alkyl of from 1 to 4 carbon atoms and may be, for example, methyl, ethyl, *n*-propyl, isopropyl, *n*-butyl or isobutyl.

As used herein, the term "a benzoyl group" is taken to mean a group of the formula $—(CO)C_6H_5$.

As used herein, the term "a straight or branched chain lower alkylcarbonyl of from 1 to 4 carbon atoms" is taken to mean a group of the structure

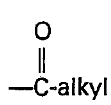

wherein the alkyl moiety is a straight or branched chain lower alkyl of from 1 to 4 carbon atoms and may be, for example, methyl, ethyl, *n*-propyl, isopropyl, *n*-butyl or isobutyl.

The preferred compounds of this invention are those compounds of Formula 1 wherein R is hydrogen, $R_1$ is methyl or ethyl and $X_1$ is at the para position and is methylsulfoxide or methylsulfone.

When R is hydrogen in Formula 1 compounds, the several tautomeric forms of general Formula 2 as possible;

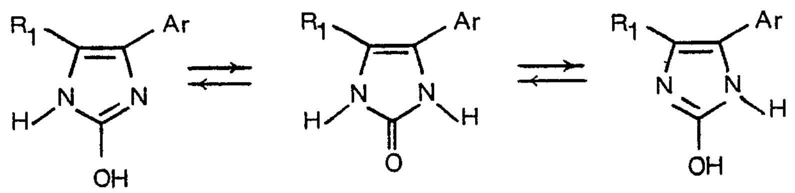

Formula 2

wherein $R_1$ and Ar are as defined in Formula 1. These acidic tautomers may form pharmaceutically active salts of general Formula 3

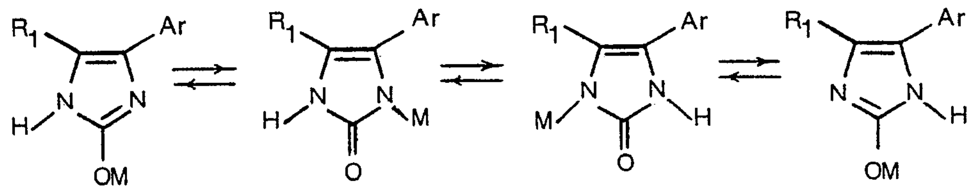

Formula 3

wherein $R_1$ and Ar are as defined in Formula 1, and M is a pharmaceutically acceptable alkali metal, such as sodium or potassium; alkaline earth metal, such as calcium or magnesium; transition metal, such as zinc or iron; main group metal; ammonium or organic ammonium ion, such as tetramethylammonium ion. Throughout this disclosure the term imidazol-2-one shall be taken to mean any of the tautomers of Formula 2 and a pharmaceutically acceptable salt of an imidazol-2-one shall be taken to mean any tautomer of Formula 3.

The 4-aroylimidazol-2-ones of this invention wherein R is hydrogen may be prepared by a Friedel-Crafts acylation of an imidazol-2-one of Formula 4:

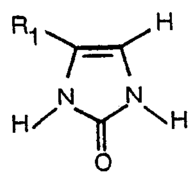

Formula 4

wherein $R_1$ is as defined in Formula 1. The acylating agent may be a benzoyl halide, preferably a benzoyl chloride, of Formula 5

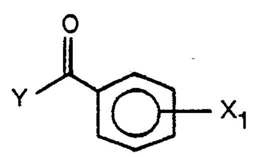

Formula 5

wherein Y is a halogen and $X_1$ is as defined in Formula 1. Furthermore, the Friedel-Crafts reaction may be performed on the free acid or its corresponding acid anhydride instead of the aroyl halides mentioned hereinabove employing essentially identical reaction conditions. These alternate reactions are more fully described in Olah, "Friedel-Crafts and Related Reactions", Vol. III, Part 1, Interscience Publications, John Wiley and Sons, New York, 1964.

The Friedel-Crafts reactions of this invention are performed by premixing about 1 molar equivalent of the appropriate imidazol-2-one with about 1 molar equivalent to about 10 molar equivalents, preferably about 2 molar equivalents, of a Lewis acid catalyst in a suitable solvent for example, petroleum ethers; a chlorinated hydrocarbon, such as carbon tetrachloride, ethylene chloride, methylene chloride or chloroform; a chlorinated aromatic, such as 1,2,4-trichlorobenzene or *o*-dichlorobenzene; carbon disulfide; or preferably nitrobenzene. About 1 molar equivalent to about 10 molar equivalents, preferably about 1.1 molar equivalents of the appopriate aroyl compound is added, preferably dropwise, to the mixture of imidazol-2-one, Lewis acid, and solvent and the reaction is allowed to proceed for about 1/2 hour to about 100 hours, preferably from about 1 hour to about 10 hours depending on the reactants, the solvent, and the temperature which can be from about −78° to about 150°C, preferably about 0° to about 100°C, most preferably about 60°C. The resulting aroylimidazol-2-one may be isolated from the reaction mixture by any suitable art-known procedure, preferably by quenching the reaction mixture with ice water and subsequently removing the product by filtration or extraction and solvent removal.

Lewis acid catalysts suitable for use in the Friedel-Crafts reactions described herein are, for example, a metal, such as aluminum, cerium, copper, iron, molybdenum, tungsten or zinc; a Bronstead acid, such as a phosphoric acid, sulfuric acid, sulfonic acid, or a hydrohalo acid, such as hydrochloric or hydrobromic acid; halogen substituted acetic acids, such as chloroacetic or trifluoroacetic acids; or a metallic halide, such as a

boron halide, zinc chloride, zinc bromide, berryl chloride, copper chloride, iron (III) bromide, iron (III) chloride, mercury (II) chloride, mercury (I) chloride, antimony bromide, antimony chloride, titanium (IV) bromide, titanium (IV) chloride, titanium (II) chloride, aluminum bromide or preferably aluminum chloride.

The $X_1$ substituents may be protected as necessary in order to improve the stability of the Formula 5 reactants or to allow for the acylation of the imidazol-2-one ring nitrogen atoms as described herein without concurrent acylation of any reactive X groups.

When desired, one or both of the nitrogen atoms of the imidazol-2-one ring may be substituted with an alkyl group by any art-known procedure. Such methods include reacting the appropriate N-unsubstituted aroylimidazol-2-one of this invention with a base and an alkylating agent in presence of an unreactive solvent. Suitable bases for this reaction can be, for example, a hydride such as sodium hydride or calcium hydride; a carbonate or bicarbonate such as sodium carbonate or sodium bicarbonate; a phenoxide such as sodium phenoxide; an alkoxide such as sodium ethoxide; or preferably a hydroxide such as sodium hydroxide. Suitable alkylating agents for this reaction are, for example, an alkyl halide such as methyl chloride, methyl bromide, or methyl iodide; or a dialkylsulfate such as dimethylsulfate. Suitable unreactive solvents are, for example, petroleum ethers; chlorinated hydrocarbons such as carbon tetrachloride, chloroform, or methylene chloride; chlorinated aromatics such as 1,2,4-trichlorobenzene, *o*-dichlorobenzene, or chlorobenzene; carbon disulfide; nitrobenzene; ethereal solvents such as diethyl ether, tetrahydrofuran or *p*-dioxan; aromatic solvents such as benzene, toluene or xylene; or preferably the polar aprotic solvents such as dimethylformamide (DMF) a dimethylsulfoxide (DMSO). The reaction is allowed to proceed from about 1 minute to about 1 hour and the temperature may be from about 0° to about 100°C, preferably about 25°C. When it is desired that only one of the imidazol-2-one nitrogen atoms be substituted with an alkyl group, the appropriate imidazol-2-one is reacted with from about 1 molar equivalent to about 10 molar equivalents of a base, preferably about 1 molar equivalent and with about 1 molar equivalent of an alkylating agent. Utilizing this procedure, both possible monoalkylated nitrogen isomers result. These isomers are separable by conventional art-known procedures such as fractional crystallization, fractional distillation, or chromatography. When it is desired that both nitrogen atoms of the imidazol-2-one ring by alkyl substituted, the appropriate imidazol-2-one is reacted with from about 2 molar equivalents to about 10 molar equivalents of a base, preferably about 2 molar equivalents and from about 2 molar equivalents to about 10 molar equivalents of an alkylating agent, preferably about 2 molar equivalents.

When desired, the nitrogen atoms of the imidazol-2-one ring may be substituted with an alkylcarbonyl group by any suitable art-known procedure. Such methods include reacting the N-substituted aroylimidazol-2-ones of this invention with an acyl halide, preferably an acyl chloride such as acetyl chloride, *n*-propanoyl chloride, isopropanoyl chloride or butanoyl chloride. Normally acylation reactions utilizing acyl halides employ an acid sponge such as triethylamine or pyridine to remove any hydrohalide as it is formed. Furthermore, the corresponding free acid or acid anhydride may be employed instead of the acyl halides. Acylation reactions are generally run without added solvent but may be performed using any nonreactive solvent, for example, petroleum ethers; chlorinated hydrocarbons such as chloroform, methylene chloride or carbon tetrachloride; carbon disulfide; ethereal solvents, such as diethylether, tetrahydrofuran or *p*-dioxan or aromatic solvents such as benzene, toluene or xylene. The reactions are allowed to proceed for about 1 minute to about 100 hours, preferably from about 1 hour to about 10 hours and the temperature may be from about −78° to about 150°C, preferably from 0° to 100°C.

Alternatively, the compounds of formula 1 wherein $X_1$ is a straight or branched chain lower alkylsulfoxide or alkylsulfone of from 1 to 4 carbon atoms may be prepared by selectively oxidizing an appropriate compound of formula 1 wherein $X_1$ is a straight or branched chain lower alkylthio of from 1 to 4 carbon atoms. Selective oxidation to produce a sulfoxide can be accomplished by reaction with 1 equivalent of hydrogen peroxide and selective oxidation to produce a sulfone can be accomplished by reaction with 2 or more equivalents of hydrogen peroxide.

The alkali metal, alkaline earth metal, transition metal, main group metal, ammonium or organic ammonium salts of the aroylimidazol-2-ones of this invention may be prepared from a corresponding metal or ammonium basic salt for example an alkoxide, such as sodium methoxide or sodium ethoxide; a phenoxide, such as sodium phenoxide; hydroxides, such as sodium hydroxide or potassium hydroxide; or a carbonate, such as sodium carbonate, potassium carbonate, zinc carbonate, magnesium carbonate or sodium hydrogen carbonate. These reactions may be performed with or without a solvent. Suitable solvents are, for example, lower alcohols, such as methanol, ethanol, isopropanol, *n*-propanol or *n*-butanol; aromatic solvents, such as benzene, toluene or xylene; ethereal solvents, such as diethyl ether, tetrahydrofuran or *p*-dioxan; and halogenated hydrocarbon solvents, such as chloroform, methylene chloride or carbon tetrachloride. The aroylimidazol-2-one and base are allowed to react for about 1 minute to about 24 hours depending on the reactants and the temperature which can be from about −78° to about 150°C, preferably from about 0° to about 25°C.

The aroyl chlorides or their corresponding carboxylic acids, which are required for the Friedel-Crafts acylation of this invention, are either generally available in the art or may be prepared by analogous procedures. The imidazol-2-one starting materials of Formula 4 may be prepared as described by or adapted from R. Duschinsky and L. A. Dolan, *J. Am. Chem. Soc. 67*, 2079 (1945), R. Duschinsky and L. A. Dolan, *J. Am. Chem. Soc. 68.* 2350 (1945) or U.S. patent 2,441,933.

The compounds of general Formula 1 may be used in the treatment of cardiac failure including congestive heart failure, backward heart failure, forward heart failure, left ventricular heart failure, or right ventricular heart failure or in the treatment of any other condition which requires the strengthening of heart action with a cardiotonic. In many respects these compounds possess digitalis-like action. The compounds of general Formula 1 may also be used in the treatment of hypertensive including primary or essential hypertension, hormonally induced hypertension, renal hypertension and chemically induced hypertension. Finally, the compounds of general Formula 1 may be used as antithrombotics. They affect the coagulation of blood by preventing the aggregation of blood platelets, which play a dominant role in thrombotic conditions both in the initial event and at the occlusive stage. Arterial thrombosis, particularly in arteries supplying the heart muscle and brain, is a leading cause of death and disability.

The compounds may be administered in various manners to achieve the desired effect. The compounds may be administered alone or in the form of pharmaceutical preparations to the patient being treated either orally or parenterally, that is, intravenously or intramuscularly. The amount of compound administered will vary with the severity of the hypertension, cardiac failure or blood clotting and the mode of administration. For oral administration the antihypertensively effective amount of compound is from about 0.1 mg/kg (milligrams per kilograms) of patient body weight per day to about 500 mg/kg of patient body weight per day and preferably from about 50 mg/kg of patient body weight per day to about 150 mg/kg of patient body weight per day.

For parenteral administration the antihypertensively effective amount of compound is from about 0.01 mg/kg of patient body weight per day up to about 150 mg/kg of patient body weight per day and preferably from about 1.0 mg/kg of patient body weight per day up to about 10.0 mg/kg of patient body weight per day. For oral or parenteral administration the cardiotonically effective amount of compound is from about 0.1 mg/kg of patient body weight per day up to about 500 mg/kg of patient body weight per day and preferably from about 0.1 mg/kg of patient body weight per day up to about 10.0 mg/kg of patient body weight per day. For oral or parenteral administration the anticoagulant effective amount of compound is from about 0.1 mg/kg of patient body weight per day up to about 1000 mg/kg of patient body weight per day and preferably from about 1 mg/kg of patient body weight per day up to about 100 mg/kg of patient body weight per day.

For oral administration a unit dosage may contain for example, from 10 to 100 mg of the active ingredient. For parenteral administration a unit dosage may contain, for example, from 5 to 50 mg of the active ingredient. Repetitive daily administration of the compounds may be desired and will vary with the condition of the patient and the mode of administration.

As used herein the term patient is taken to mean a warm blooded animal, for example, birds, such as chickens and turkeys, and mammals, such as primates, humans, sheep, horses, bovine cows and bulls, pigs, dogs, cats, rats, and mice.

For oral administration the compounds can be formulated into solid or liquid preparations such as capsules, pills, tablets, troches, powders, solutions, suspensions or emulsions. The solid unit dosage forms can be a capsule which can be of the ordinary gelative type containing, for example, lubricants and inert filler, such as lactose, sucrose, and cornstarch. In another embodiment the compounds of general Formula 1 can be tableted with conventional tablet bases such as lactose, sucrose, and cornstarch in combination with binders, such as acacia, cornstarch or gelatin, disintegrating agents such as potato starch or alginic acid, and a lubricant such as stearic acid or magnesium stearate.

For parenteral administration the compounds may be administered as injectable dosages of a solution or suspension of the compound in a physiologically acceptable diluent with a pharmaceutical carrier which can be a sterile liquid such as water and oils with or without the addition of a surfactant and other pharmaceutically acceptable adjuvants. Illustrative of oils which can be employed in these preparations are those of petroleum, animal, vegetable, or synthetic origin, for example, peanut oil, soybean oil, and mineral oil. In general, water, saline, aqueous dextrose and related sugar solutions, ethanol and glycols such as propylene glycol or polyethylene glycol are preferred liquid carriers, particularly for injectable solutions.

The compounds can be administered in the form of a depot injection or implant preparation which may be formulated in such a manner as to permit a sustained release of the active ingredient. The active ingredient can be compressed into pellets or small cylinders and implanted subcutaneously or intramuscularly as depot injections or implants. Implants may employ inert materials such as biodegradable polymers or synthetic silicones, for example, Silastic, silicone rubber manufactured by the Dow-Corning Corporation.

The following specific examples further illustrates the preparation of compounds employed in the instant invention.

Example 1

1,3-Dihydro-5-Methyl-4-(4-methylsulfinylbenzoyl)-2*H*-imidazol-2-one

To 20 g of 1,3-dihydro-5-methyl-4-[4-(methylthio)-benzoyl]-2*H*-imidazol-2-one in glacial acetic acid is added 11.3 g (1.25 equivalents) of 30% hydrogen peroxide. The solution is warmed at 50°C for 2½ hours and then allowed to cool overnight. Acetic acid is removed *in vacuo* to leave the title compound, m.p. 276—278°C (dec.).

Example 2

1,3-Dihydro-5-Methyl-4-(4-methylsulfonylbenzoyl)-2*H*-imidazol-2-one

To 25 g of 1,3-dihydro-5-methyl-4-[4-(methylthio)-benzoyl]-2*H*-imidazol-2-one in glacial acetic acid is added 34 g (3 equivalents) of 30% hydrogen peroxide. The solution is warmed at 50°C for 20 hours. Acetic acid is removed *in vacuo* to leave the title compound, m.p. 315—318°C (dec.).

Example 3

1,3-Dihydro-5-ethyl-4-(4-methylsulfonylbenzoyl)-2*H*-imidazol-2-one

In 100 g polyphosphoric acid is placed 2.0 g *p*-(methylsulfonyl)benzoic acid and 1.12 g 1,3-dihydro-5-ethyl-2*H*-imidazol-2-one. The mixture is heated to 150°C for 5 hours, cooled and quenched with 1000 ml of water. The title compound, which separates as a solid, is collected and washed with water.

By substituting 4-(methylsulfinyl)benzoic acid for 4-(methylsulfonyl)benzoic acid in the above procedure, the compound 1,3-dihydro-5-ethyl-4-(4-methylsulfinylbenzoyl)-2*H*-imidazol-2-one is obtained.

**Claims**

1. A compound of the formula

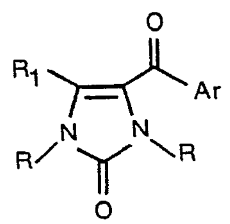

wherein Ar is phenyl monosubstituted at the ortho, meta or para position with $X_1$; $X_1$ is a straight or branched chain lower alkylsulfoxide of from 1 to 4 carbon atoms or a straight or branched chain lower alkylsulfone of from 1 to 4 carbon atoms; R is hydrogen, a straight or branched chain lower alkyl of from 1 to 4 carbon atoms, a straight or branched chain lower alkylcarbonyl of from 1 to 4 carbon atoms, or a benzoyl; $R_1$ is hydrogen or a straight or branched chain lower alkyl of from 1 to 4 carbon atoms or a pharmaceutically acceptable salt thereof.

2. A compound of claim 1 wherein R is hydrogen.

3. A compound of claim 1 wherein R is hydrogen and $R_1$ is hydrogen, methyl or ethyl.

4. A compound of claim 3 wherein $X_1$ is at the para position.

5. A compound of claim 3 wherein $X_1$ is a methylsulfoxide or methylsulfone group.

6. A compound of claim 4 wherein $R_1$ is methyl and $X_1$ is a methylsulfoxide.

7. A compound of claim 4 wherein $R_1$ is methyl and $X_1$ is a methylsulfone.

8. A process for preparing the aroylimidazol-2-ones of claim 1 which consists of a Friedel-Crafts acylation of a compound of the formula

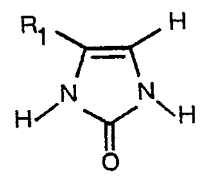

wherein $R_1$ is as defined in claim 1, with about 1 to about 10 molar equivalents of a benzoyl halide monosubstituted at the ortho, meta, or para position with $X_1$ as defined in claim 1, in the presence of about 1 to about 10 molar equivalents of a Lewis acid catalyst in a suitable solvent at a temperature of from 0° to about 100°C for about 1 to about 10 hours; when it is desired that R be other than hydrogen, acylating or alkylating, as appropriate, the resulting aroylimidazol-2-one with an appropriate acyl halide or alkylating agent; and where a pharmaceutically acceptable salt is desired, reacting the thus formed aroylimidazol-2-one with an appropriate metal or ammonium basic salt.

9. A process for preparing the aroylimidazol-2-ones of claim 1 which consists of selectively oxidizing a compound of the formula

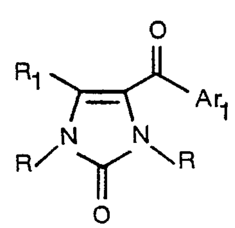

wherein R and $R_1$ are as defined in claim 1 above and $Ar_1$ is a benzoyl halide monosubstituted in the ortho, meta, or para position with a straight or branched chain lower alkylthio of from 1 to 4 carbon atoms as appropriate and where a pharmaceutically acceptable salt is desired, reacting the thus formed aroylimidazol-2-one with an appropriate metal or ammonium basic salt.

10. Pharmaceutical composition useful as an antihypertensive, cardiotonic or antithrombotic agent, characterized by a content of a compound according to Claims 1 to 8 and usual carriers, adjuvants and additives.

11. Use of the compounds of Claims 1 to 8 for preparing a pharmaceutical composition according to Claim 10.

**Patentansprüche**

1. Verbindungen der Formel

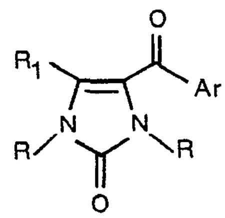

in der Ar eine in ortho-, meta- oder para-Stellung mit $X_1$ monosubstituierte Phenylgruppe bedeutet, $X_1$ einen unverzweigten oder verzweigten Niederalkylsulfoxidrest mit 1 bis 4 Kohlenstoffatomen oder einen unverzweigten oder verzweigten Niederalkylsulfonrest mit 1 bis 4 Kohlenstoffatomen darstellt, R ein Wasserstoffatom, einen unverzweigten oder verzweigten Niederalkylrest mit 1 bis 4 Kohlenstoffatomen, einen unverzweigten oder verzweigten Niederalkylcarbonylrest mit 1 bis 4 Kohlenstoffatomen oder eine Benzoylgruppe bedeutet, $R_1$ ein Wasserstoffatom oder einen unverzweigten oder verzweigten Niederalkylrest mit 1 bis 4 Kohlenstoffatomen darstellt, oder pharmazeutisch verträgliche Salze davon.

2. Verbindungen nach Anspruch 1, wobei R ein Wasserstoffatom bedeutet.

3. Verbindungen nach Anspruch 1, wobei R ein Wasserstoffatom und $R_1$ ein Wasserstoffatom, eine Methyl- oder Äthylgruppe bedeuten.

4. Verbindungen nach Anspruch 3, wobei sich $X_1$ in para-Stellung befindet.

5. Verbindungen nach Anspruch 3, wobei $X_1$ eine Methylsulfoxid- oder Methylsulfongruppe bedeutet.

6. Verbindungen nach Anspruch 4, wobei $R_1$ eine Methylgruppe und $X_1$ eine Methylsulfoxidgruppe bedeuten.

7. Verbindungen nach Anspruch 4, wobei $R_1$ eine Methylgruppe und $X_1$ eine Methylsulfongruppe bedeuten.

8. Verfahren zur Herstellung der Aroylimidazol-2-one nach Anspruch 1, bestehend aus einer Friedel-Crafts-Acylierung einer Verbindung der Formel

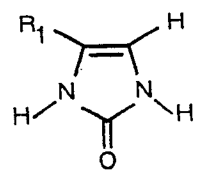

in der $R_1$ wie in Anspruch 1 definiert ist, mit etwa 1 bis 10 Moläquivalenten eines in ortho-, meta- oder para-Stellung mit $X_1$ monosubstituierten Benzoylhalogenids gemäß Anspruch 1, in Gegenwart von etwa 1 bis 10 Moläquivalenten eines Lewissäuren-Katalystors in einem geeigneten Lösungsmittels bei einer Temperatur von etwa 0 bis 100°C für etwa 1 bis 10 Stunden; wenn R eine andere Bedeutung als Wasserstoff haben soll, je nachdem Acylierung oder Alkylierang des erhaltenen Aroylimidazol-2-ons mit einem entsprechenden Acylhalogenid oder Alkylierungsmittel; und wenn ein pharmazeutisch verträgliches Salz gewünscht wird, Umsetzung des so entstandenen Aroylimidazol-2-ons mit einem entsprechenden basischen Metall- oder Ammoniumsalz.

9. Verfahren zur Herstellung der Aroylimidazol-2-one nach Anspruch 1, bestehend aus der selektiven Oxidation einer Verbindung der Formel

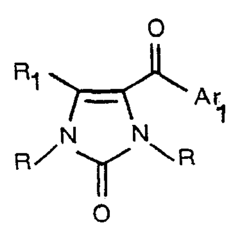

in der R und $R_1$ wie in Anspruch 1 definiert sind und $Ar_1$ ein in ortho-, meta- oder para-Stellung mit einem unverzweigten oder verzweigten Niederalkylthiorest mit 1 bis 4 Kohlenstoffatomen monosubstituiertes Benzoylhalogenid darstellt, wie erforderlich, und wenn ein pharmazeutisch verträgliches Salz gewünscht wird, Umsetzung des so entstandenen Aroylimidazol-2-ons mit einem entsprechenden basischen Metall- oder Ammoniumsalz.

10. Als Antihochdruckmittel, cardiotonisches oder antithrombotisches Mittel nützliches Arzneimittel, gekennzeichnet durch einen Gehalt an einer Verbindung nach den Ansprüchen 1 bis 8 und übliche Träger-, Hilfs- und Zusatzstoffe.

11. Verwendung der Verbindungen nach Anspruch 1 bis 8 zur Herstellung eines Arzneimittels nach Anspruch 10.

**Revendications**

1. Un composé de formule

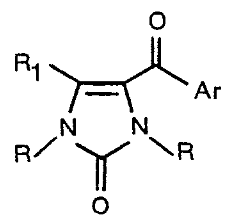

où Ar est un phényle monosubstitué sur la position ortho, méta ou para avec $X_1$; $X_1$ est un alkylsulfoxyde inférieur à chaîne droite ou ramifiée de 1 à 4 atomes de carbone ou une alkylsulfone inférieure à chaîne droite ou ramifiée de 1 à 4 atomes de carbone; R est un hydrogène, un alkyle inférieur à chaîne droite ou ramifiée de 1 à 4 atomes de carbone, un alkylcarbonyle inférieur à chaîne droite ou ramifiée de 1 à 4 atomes de carbone ou un benzoyle; $R_1$ est un hydrogène ou un alkyle inférieur à chaîne droite ou ramifiée de 1 à 4 atomes de carbone ou un sel convenant en pharmacie correspondant.

2. Un composé selon la revendication 1 où R est un hydrogène.

3. Un composé selon la revendication 1 où R est un hydrogène et $R_1$ est un hydrogène, un méthyle ou un éthyle.

4. Un composé selon la revendication 3 où $X_1$ est un position para.

5. Un composé selon la revendication 3 où $X_1$ est un groupe méthylsulfoxyde ou méthylsulfone.

6. Un composé selon la revendication 4 où $R_1$ est un méthyle et $X_1$ est un méthylsulfoxyde.

7. Un composé selon la revendication 4 où $R_1$ est un méthyle et $X_1$ est un méthylsulfone.

8. Un procédé pour la préparation des aroylimidazole-2-ones de la revendication 1 qui consiste en une acylation de Friedel-Crafts d'un composé de formule

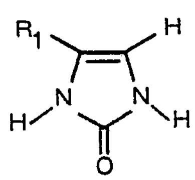

ou $R_1$ est comme défini dans la revendication 1, avec environ 1 à environ 10 équivalents molaires d'un halogénure de benzoyle monosubstitué sur la position ortho, métha ou para avec $X_1$ comme défini dans la revendication 1, en présence d'environ 1 à environ 10 équivalents molaires d'un catalyseur constitué d'un acide de Lewis dans un solvant approprié à une température d'environ 0° à environ 100°C pendant environ 1 à environ 10 heures; lorsqu'on désire que R soit autre qu'un hydrogène, l'acylation ou l'alkylation, comme il convient, de l'aroylimidazole-2-one obtenue avec un halogénure d'acyle ou un agent d'alkylation appropriés; et lorsqu'on désire un sol convenant en pharmacie la réaction de l'aroylimidazole-2-one ainsi formée avec un sel basique approprié de métal ou d'ammonium.

9. Un procédé pour la préparation des aroylimidazole-2-ones de la revendication 1 qui consiste en l'oxydation sélective d'un composé de formule

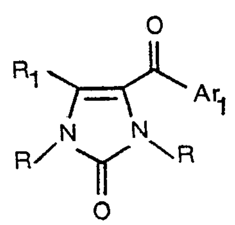

où R et $R_1$ sont comme défini dans la revendication 1 ci-dessus et $Ar_1$ est un halogénure de benzoyle monosubstitué dans la position ortho, méta or para avec un alkylthio inférieur à chaîne droite ou ramifiée de 1 à 4 atomes de carbone comme il convient et où, lorsqu'on désire un sel convenant en pharmacie, la réaction de l'aroylimidazole-2-one ainsi formée avec un sel basique approprié de métal ou d'ammonium,.

10. Une composition pharmaceutique utile comme agent antihypertenseur, cardiotonique ou antithrombotique caractérisée en ce qu'elle contient un composé selon les revendications 1 à 8 et des supports, adjuvants et additifs habituels.

11. L'emploi des composés des revendications 1 à 8 pour la préparation d'une composition pharmaceutique selon la revendication 10.